# EUROPEAN PATENT SPECIFICATION

(11) **EP 0 461 333 B1**
(45) Date of publication and mention of the grant of the patent: **29.03.1995**
(21) Application number: 90830261.5
(22) Date of filing: 11.06.1990
(51) Int. Cl.: A61K 9/127, A61K 7/00

(54) **Phospholipidic liposomes containing active principles and a process for the production thereof**
Aktive Bestandteile enthaltende phospholipidische Liposome und Verfahren zu ihrer Herstellung
Liposomes phospholipidiques contenant des principes actifs et leur procédé de préparation

(43) Date of publication of application: 18.12.1991
(73) Proprietor: IDI FARMACEUTICI S.P.A., I-00040 Pomezia RM (IT)
(72) Inventor: Guarnieri, Decimo, I-00040 Pomezia RM (IT); Sarti, Paolo, I-00152 Roma RM (IT)
(74) Representative: Bazzichelli, Alfredo

(56) References cited:
- EP-A- 0 107 559
- EP-A- 0 241 573
- EP-A- 0 332 478
- EP-A- 0 349 429
- FR-A- 2 472 384
- FR-A- 2 591 105

## Description

The present invention relates to a process for the production of phospholipidic liposomes containing hydrophilic and hydrophobic active principles and to the obtained products.

One of the topical purposes of cosmetics nowadays is not only an improvement in the appearance of our body but also an improvement in our health. If we focus our observations to the cutis, for instance, we notice it can be subject to alterations which can have either a "physiological" origin (such as aging) or a "pathological" origin. The origin of these alterations may be internal or external to our body, the latter having mainly a physico-chemical origin related to the environment we live in. Stressful environmental conditions (intense cold or heat), variations in relative humidity or in ventilation, such as exposure to solar rays or to atmospheric chemical pollutants are clear examples. Dermatologic cosmetics is no longer limited to simple aesthetical interventions but is increasingly pursuing the aim of better protecting the cutis, particularly in re-establishing the status of hydration and elasticity (trophism) which is necessary for its optimal functioning. When the above referred alterations affect mainly the outermost layer of the cutis, the epidermis, in order to re-establish its functional status, local applications of substances which can counterbalance the negative effect of aggressive agents are carried out. Therefore dry skins are treated with fatty creams, that is those containing a high percentage of natural oils, neutral fats or neutralized saturated fatty acids. Where dryness is mainly determined by dehydration, the moisturising creams used contain substances which can absorb water (saccharopolymers or polyoxyethylenic polymers) which then transfer the water to the cutis after application. However, one of the most complex problems in the formulation of a cream for dermatologic cosmetics is however the introduction of active principles showing, for instance, chemical instability or low solubility limits, high hydrophobicity or hydrophilicity and so forth. This difficulty increases when such compounds must reach the deepest layers of the cutis (the "derma") to improve its vital conditions. As a matter of fact the derma is responsible for maintaining the elasticity and proper functioning of the outermost layers. It has already been described in scientific literature what difficulties exist for very hydrophilic substances to reach the derma, which can be explained considering that our skin is mainly waterproof. In the cosmetic formulations known from the state of art in order to reach the derma with more hydrophilic compounds, it has been necessary to make compromises, such as the removal of the outermost lipophilic barrier by means of organic solvents or by the introduction of the hydrophilic compounds in carriers containing emulsifiers or surface active agents such as Tween or Triton.

It has been now found, surprisingly, that liposomes can play a new role in allowing highly hydrophilic substances to reach the derma. Liposomes are microscopical blisters having a variable size (starting from some nanometers to some micrometers) and are artificially formed, mostly from phospholipidic mixtures which are the same phisiological components of the animal or vegetal cell membranes. The importance of these blisters relates to their tridimensional structure which, in turn, relates to the particular chemical composition of its components, the phospholipids. These biological compounds, having a lipidic origin, are substantially made of glycerin esters where an hydroxy group has been esterified by an ortophosphoric group and the other two hydroxy groups are esterified by fatty acids having chains of variable length which can also be branched. The phosphoric moiety is characterized by being electrostatically charged and it is called polar head; said polar head is therefore highly hydrophilic while the hydrocarbon chains are highly hydrophobic. In an aqueous environment and as a function of their concentration with regard to the solvent, these compounds spontaneously form blister structures where the polar head faces other polar heads (multiple layers) or the water, while the hydrophobic regions in turn face one another so as to repel water. The liposomes are uni- or multilamellas stuctures where one or more double concetric layers are formed, each double layer being in turn formed by two single layers where the polar heads are turned towards the hydrophilic phase, while the hydrophobic tails are in mutual contact so that after the liposome has formed the water can not enter the liposome. A very interesting application of the liposomes is that according which both the hydrophobic substances (in the layer between hydrophobic tails) and the hydrophilic substances (in the water volume entrapped within the liposome as between concentric lamellas) may be brought about by the same liposome. Moreover it is to be stressed that the chemical (natural) origin of phospholipids from biological membranes makes it possible for them to be re-absorbed from the epidermis into the deepest layers.

It has already been proposed in the state of art to use such structures in view of their biological origin which shows the great advantage of non-toxicity. However the processes making use of such structures have encountered drawbacks of a twofold nature, which made difficult the application of same structures:
a) the phospholipids which are commonly used to form liposome with fixed characteristics (shape, size, internal volumes, number of layers and so on) must be handled in physico-chemical conditions which are compatible with their biological origin and therefore at temperatures between 20 to 37 °C and pH between 6 to 8;
b) after their formation, particularly in the case of multilamellar liposomes, they are very stable and as a consequence, it will be very difficult to add new compounds or active principles. These compounds or active principle, particularly in case of hydrophobic substances, find an almost insuperable obstacle in the outermost polar heads exposed to water; in case of hydrophilic compounds and/or active principles, also considering their charge and their degree of ionization, they will mostly adhere electrostatically to the polar heads of the outermost layer.

In view of the above stated difficulties in the state of the art, liposomes are used in cosmetics only additioned as preformed co-principles, to the cosmetic mixtures or as carriers of hydrolized animal elastin and of total hydrophilic extracts of spleen (oxydermin).

EP-A-0349429 discloses a process for the production of llposomes in which a substantially ethanolic phase containing a lipophilic substance and eventually a lipophilic active principle is added to a substantially aqueous phase eventually containing a hydrophilic active principle.

EP-A-107559 discloses a process for the homogeneisation of dispersions of already formed liposomes by feeding under pressure the dispersions through an orifice of variable size.

Subject of the present invention is a process for the production of phospholipidic liposomes in suspension in an aqueous solvent at room temperature containing hydrophilic and/or hydrophobic active principles characterized by the fact of comprising the following steps:
a) solubilization of hydrophilic active principles in said aqueous solvent;
b) homogeneous dispersion of hydrophobic active principles in said aqueous solvent containing said hydrophilic active principles and
c) addition of phospholipids at concentrations between 40:1 and 10:1, referring to the weight of the added active principles, so that after 1-4 hours phospholipidic liposomes containing said active principles are formed.

In respect to the state of art it is to be emphasized that the liposomes are formed in the milieu which contains in a solubilized or dispersed form, all the active principles to be used, in this way overcoming one of the problems of the state of the art, that is the introduction of the active principle or principles inside an already formed liposome.

Among the active principles which can be carried within the liposomes according to the present invention, ialuronic acid, which is used as a normalizer for the collagen structures of the derma, and coenzyme Q10 (ubiquinone), are to be particularly considered in view of the particular role they play in cosmetic applications. Coenzyme Q10 is a physiological redox exchanger which is present in the mitochondrial respiratory chain of our cutis cells. Moreover, coenzyme Q10 has the double advantage of not only being a necessary physiological component of our cutis, but also, as a consequence of its perfect solubility in the hydrophobic liposome chains and of its redox properties, a very good stabilizer of the same liposome structure. In fact, coenzyme Q10 keeps constant the level of optimal unsaturation of the double bonds carbon-carbon in the phospholipidic hydrocarbon chains.

It goes without saying that according to the present invention other active principles which may or may not have a cosmetic importance, can be carried; among these hydrolized nucleic acids, catalase, superoxidodismutase, fitotherapic extracts and organ extracts, and minoxidil can be cited.

In order to improve the ease of application of the suspensions of phospholipidic liposomes according to the present invention containing active principles, it is also possible to add a further step to the production process (this is also part of the present invention) in which a thickening agent is added in order to produce a cream containing said phospholipidic liposomes which in turn contain the active principles.

In the process according to the present invention the ratio between phospholipids and active principles will vary from 40:1 to 10:1, preferably 12:1, and the time necessary for the formation of liposomes will be of 1-4 hours, preferably 2 hours. The process is carried out at room temperature preferably between 20 to 37 °C and a pH between 6 to 8. A further object of the present invention is the use in cosmetics of phospholipidic liposomes according to the present invention for the transport of active principles.

In the following examples the production of liposomes according to the present invention will be listed, which are used in the production of two anti-wrinkle creams and a moisturizing cream.

### Example 1

### Production of a first anti-wrinkle cream

The active principles used are: coenzyme Q10 (Esperis Spa-M);
vitamin A (hydrodispersed solution, vitamin A 100.000 IU/g, Istituto delle Vitamine - Milano);
vitamin E (DL-alpha-tocopherol, Merck Bracco Milano) purified soya-lecitins (Sigma Chemical Co. St. Louis, USA);
oxydermin (proteic extract of spleen, Sederma Variati - Milano).

### Preservers (antibacterial, antimycotic agents)

Prevan (hydroxyethyliden-methyl-pirandion, dehydroacetate sodic, NIPA - Genova);
Methylparaben (methyl-paraoxy-benzoate, NIPA - Genova);
Propilparaben (propil-paraoxy-benzoate, NIPA - Genova);
glycerin (Merck Bracco - Milano)
Germall 115 (imidazolin-urea AGRAR - Roma)

### Carriers and solubilizing phases

phosphate buffer 10 M pH 7,0 (Merck Bracco - Milano);
corn germ oil (Esperis Spa - Milano)

### Thickening agents

Carbopol 934 (neutralized carboxymethylpolimer Biochim - Milano).

Two reacting mixtures are prepared into separated 50 1 stainless steel containers at 20 °C.
A) Formation of liposomes: 0.05 kg of Q10, 300 mIU of vitamin A together with 0.1 kg of vitamin E are dissolved in 1 kg of corn germ oil. Such a homogeneous oil solution is emulsified at 25-30 g through agitation (emulsifier equipped with a stirrer, Totalia 150, Mambretti - Milano) in 44.6 kg of phosphate buffer solution 10 mM pH 7.0; 1.25 kg of purified granulated soya-lecitins are added to the solution under continuous agitation. The resulting suspension is left under uniform agitation for a period between 2 to 4 hours at a temperature of 20 °C. This time is necessary for the formation of liposomes and the presence of active principles. The final suspension is kept at 4°C in a stainless steel container before its use in the process of production of the cream.
B) Formation of the support gel: 0.01 kg of Prevan, 0.1 kg of propilparaben, 0.1 kg of methylparaben, 3.0 kg of glycerin, 0.2 kg of Germall 115, 3.0 kg of oxydermin are dissolved in 43.0 kg of a neutralized solution of Carbopol 934 (1 %). During the process the emulsifier equipped with a stirrer referred to above is used; the carboxymethylpolimer (Carbopol 934) has been selected for its water absorbtion properties; as a consequence a thickening effect occurs without any solubilization effect (cleansing) on the liposome structures formed. As a function of the required thickness, from 0.5 to 2.0 kg of neutralized carboxymethylpolimer are added. The thickened solution is kept in a 50 1 stainless steel container at room temperature before use.
C) Formation of the cream
   Two phases A and B are sucked in one after the other in the emulsifier referred to above. The mixing takes place in the following conditions: mixture A is sucked in first; then mixture B is sucked in under quick agitation (about 1000 rpm) at a temperature of 20 °C and a pressure of 400 mmHg. The process lasts about 60 minutes in conditions referred to above. At this stage the cream is perfumed with essence (Crema test Ciprosia, Dragogo - GDR Milano).

### Example 2

### Production of a second anti-wrinkle cream

Following the procedure indicated in example 1, but dissolving 0.01 kg of ialuronic acid under the form of a sodic salt in the 10 mM a phophate buffer solution at pH 7.0 before of the emulsion step and before the addition of the granulated purified soya-lecitins, a new formulation of an anti-wrinkle cream containing both the co-enzyme Q10 and the ialuronic acid is obtained.

### Example 3

### Production of a moisturizing cream

### Starting material used

### Active principles

Collastin (hydrolized animal elastin, CRODA - Milano)
vitamin A (hydrodispersed solution (vitamin A, 100,000 IU/g, Istituto delle Vitamine - Milano)
vitamin E (DL-alpha-tocopherol, Merck Bracco - Milano)
purified soya lecitins (Sigma Chem. Co. St. Louis - USA)
alpha-bisabol (active principle of chamomile, Dragogo GDR - Milano)
ialuronic acid (Lambrokim - Milano)

### Preservers (antibacterial - antimycotal agents):

Bronopol (bromonitropropandiol Formenti - Milano)
methylparaben (methyl-paraoxy-benzoate, NIPA - Genova)
Propilparaben (propil-paraoxybenzoate, NIPA - Genova)

### Carriers and solubilizing phases

Phosphate buffer 10 mM pH 7.0

### Thickening agents

Carbopol 934 (neutralized carboxymethylpolimer, Biochim - Milano)

### Methodologic sequence

Two reactive mixtures into two different 50 1 stainless steel containers at 20 °C are prepared.
A) Formation of the liposomes: 300 mIU of vitamin A, together with 0.1 kg of vitamin E, 0.02 kg of ialuronic acid and 1 kg of Collastin are dissolved in 45.3 kg of 10 mM phosphate buffer solution at pH 7.0 in the emulsifier of example 1. After the solubilization, 0.6 kg of granulated purified soya-lecitins are added under continuous agitation to the mixture. The resulting suspension is kept under uniform agitation for a period between 2 to 4 hours at a temperature of 20 °C. The final suspension is kept at 4 °C in a 50 1 stainless steel container before the process of cream production.
B) Formation of the support gel: 0.1 kg of Prevan, 0.1 kg of Propilparaben, 0.1 kg of Methylparaben, 3.0 kg of glycerin, 0.2 kg of Bronopol are dissolved in 46 kg of a neutralized solution of Carbopol 934 (1 %). According to the required density from 0.5 to 2.0 kg of neutralized carboxymethylpolymer are added. The thickened solution is kept in a 50 1 stainless steel container at room temperature before use.
C) Formation of the cream
   The two phases A and B are sucked in one after the other in the emulsifier of example 1. The mixing takes place in the following conditions:
   as a first step mixture A is sucked in; then mixture B is sucked in under quick agitation (about 1000 rpm) at a temperature of 20 °C and a pressure of 400 mmHg. The process lasts about 60 minutes in conditions referred to above. The cream is then perfumed with "Crema test Ciprosia" essence.

It must be emphasized that solubilization or monodispersion of the active principle in aqueous solvents is a function of the coefficient of hydrophobic/hydrophilic ripartition. Upon formation of the liposomes the active principles will be distributed in different percentages within the hydrophobic layers or in the zone the water is found. These active principles, particularly for the cosmetic treatment, are vitamin complexes A, and E, hydrolized animal elastin, ialuronic acid, co-enzyme Q10 and so forth. This list is to be considered only as examplificative and non-limiting, because different active principles can be introduced before application with the liposomes of the present invention.

## Claims

1. A process for the production of phospholipidic liposomes in suspension in an aqueous solvent at room temperature and containing hydrophilic and hydrophobic active principles, characterized by the fact of comprising the following steps:
A) solubilization of said hydrophilic active principles in said aqueous solvent;
B) homogeneous dispersion of the hydrophobic active principles in said aqueous solvent containing said hydrophilic active principles, and
C) addition of phospholipids to concentrations between 42:1 to 10:1 with reference to the weight of the added active principles,
so that after 1-4 hours phospholipidic liposoms containing said active principles are obtained.

2. The process according to claim 1, further comprising the step of adding a thickening agent compatible with the phospholipidic liposomes to be obtained in order to produce a cream containing said phospholipidic liposomes which contain said hydrophilic and hydrophobic active principles.

3. Cream obtained from the process as claimed in claim 2.

4. Cream according to claim 3, wherein said active principles are selected from the group comprising ialuronic acid, co-enzyme Q10 and their mixtures.

5. Cream according to claim 4, wherein said liposomes further contain active principles selected from the group formed by vitamin complexes A, vitamin complexes E, oxydermin, alphabisabol and their mixtures.

## Patentansprüche

1. Verfahren zur Herstellung von phospholipidischen Liposomen in einer Suspension in einem wäßrigen Lösungsmittel bei Raumtemperatur und enthaltend hydrophile und hydrophobe aktive Bestandteile, **dadurch gekennzeichnet**, daß es die folgenden Schritte umfaßt:
A) Solubilisation der hydrophilen aktiven Bestandteile in dem wäßrigen Lösungsmittel;
B) homogene Dispersion der hydrophoben aktiven Bestandteile in dem wäßrigen Lösungsmittel, das die hydrophilen aktiven Bestandteile enthält, und
C) Zugabe von Phospholipiden in Konzentrationen zwischen 42:1 bis 10:1 bezogen auf die Masse der zugesetzten aktiven Bestandteile,
so daß nach ein bis vier Stunden phospholipidische Liposome erhalten werden, die diese aktiven Bestandteile enthalten.

2. Verfahren nach Anspruch 1, zusätzlich den Schritt enthaltend, daß ein Verdickungsmittel zugegeben wird, das mit den erhaltenen phospholipidischen Liposomen verträglich ist, um eine Creme herzustellen, die diese phospholipidischen Liposome enthält, die die hydrophilen und hydrophoben aktiven Bestandteile enthalten.

3. Creme erhalten nach dem Verfahren nach Anspruch 2.

4. Creme nach Anspruch 3, wobei die aktiven Bestandteile ausgewählt werden unter Hyaluronsäure, Co-Enzym Q10 und deren Gemische.

5. Creme nach Anspruch 4, wobei die Liposome zudem aktive Bestandteile ausgewählt unter Vitamin-A-Komplexen, Vitamin-E-Komplexen, Oxydermin, Alphabisabol und deren Gemische enthalten.

## Revendications

1. Procédé pour la production de liposomes phospholipidiques en suspension dans un solvant aqueux à température ambiante et contenant des principes actifs hydrophobes et hydrophiles, caractérisé par le fait qu'il comprend les étapes suivantes :
A) Solubilisation des dits principes actifs hydrophiles dans le dit solvant aqueux ;
B) Dispersion homogène des principes actifs hydrophobes dans le dit solvant aqueux contenant les dits principes actifs hydrophiles, et
C) Addition de phospholipides à une concentration comprise entre 42:1 et 10:1 par rapport au poids des principes actifs ajoutés, de telle sorte qu'après 1 à 4 heures on obtient des liposomes phospholipidiques contenant les dits principes actifs.

2. Procédé selon la revendication 1, comprenant en outre l'étape d'addition d'un agent épaississant compatible avec les liposomes phospholipidiques à obtenir, pour produire une crème contenant les dits liposomes phospholipidiques qui contiennent les dits principes actifs hydrophobes et hydrophiles.

3. Crème obtenue selon le procédé de la revendication 2.

4. Crème selon la revendication 3, dans laquelle les dits principes actifs sont choisis à partir du groupe comprenant de l'acide ialuronique, le co-enzyme Q10 et leurs mélanges.

5. Crème selon la revendication 4, dans laquelle les dits liposomes contiennent en outre des principes actifs choisis dans le groupe formé par les complexes de vitamines A, complexes de vitamines E, l'oxydermine, l'alphabisabol, et leurs mélanges.
